# EUROPÄISCHE PATENTSCHRIFT

(11) **EP 0 652 013 B1**
(45) Veröffentlichungstag und Bekanntmachung des Hinweises auf die Patenterteilung: **19.08.1998**
(21) Anmeldenummer: 94116701.7
(22) Anmeldetag: 22.10.1994
(51) Int. Cl.: A61K 35/20

(54) **Verwendung boviner Kolostralmilch zur Herstellung eines Arzneimittels für die Behandlung von Leberfunktionsstörungen**
Use of bovine colostrum for the manufacture of a medicament for the treatment of liver malfunctions
Utilisation de colostrum bovin pour la manufacture d'un médicament pour le traitement des perturbations fonctionelles du foie

(30) Priorität: 04.11.1993 DE 4337654
(43) Veröffentlichungstag der Anmeldung: 10.05.1995
(73) Patentinhaber: BIOTEST PHARMA GMBH, D-63303 Dreieich (DE)
(72) Erfinder: Möller, Wolfgang, Dr., D-61440 Oberursel (DE); Lissner, Reinhard, Dr., D-63937 Gönz-Weilbach (DE); Nitsche, Dietrich, Dr., D-24105 Kiel (DE)
(74) Vertreter: Beil, Hans Chr., Dr.

(56) Entgegenhaltungen:
- EP-A- 0 471 890

## Beschreibung

Die Erfindung betrifft die Verwendung von unter Keimarmen Bedingungen gewonnener Kolostralmilch mit nativen Proteinen oder Kolostralmilch-Zubereitungen vom Rind oder daraus hergestellter Immunglobulin-haltiger Fraktionen zur Herstellung eines Leberschutzpräparates zur Vorbeugung und Behandlung von Leberfunktionsstörungen bei Erkrankungen der Leber, insbesondere der Vermeidung der Folgen dieser Erkrankungen, wie z. B. der portalen Enzephalopathie.

Die Leber hat vielfältige Funktionen im Stoffwechsel des Menschen. Zu ihren Aufgaben gehört unter anderem die Bildung und Ausscheidung der Galle, die Umwandlung von Monosacchariden in Glykogen, die Glykogenolyse, die Glukoneogenese aus Proteinen, der Fettsäureabbau, die Verwertung und Desaminierung der Aminosäuren, die Bildung von Plasmaproteinen sowie die Entgiftung und Ausscheidung toxischer Stoffwechselprodukte. Ein besonders toxisches Stoffwechselprodukt ist Ammoniak, das einmal bei dem bakteriellen Abbau von Proteinen im Darm und bei dem Abbau der Aminosäuren in der Leber selbst entsteht. Ammoniak wird in der gesunden Leber über den Harnstoffzyklus zu Harnstoff abgebaut oder über Glutaminsäure entgiftet.

In den industrialisierten Länder sind mindestens 1 bis 2 % der Bevölkerung chronisch an einer Leberinsuffizienz auf Grund eines Leberparenchymschadens erkrankt. Neben den akuten und chronischen Hepatitiden treten hierbei u.a. die Fettleber und die Leberzirrhose hervor.

Die Hepatitiden umfassen alle hepatozelluläre Erkrankungen, die mit einer entzündlichen Reaktion des Lebergewebes auf Grund von z.B. Virus- und Bakterieninfektionen oder Arzneimittel- oder Alkoholvergiftungen zusammenhängen. Ein Teil der akuten Hepatitiden gehen in eine chronische Hepatitis über, die wiederum zu einer Leberzirrhose führen können.

Bei der Leberzirrhose handelt es sich um eine chronische Lebererkrankung, bei der es u.a. nach Parenchymnekrosen zu einem Parenchymumbau mit einer Vermehrung des Bindegewebes kommt. Neben Virushepatitiden ist der chronische Alkoholmißbrauch die Hauptursache für das Auftreten der Leberzirrhose.

Man unterscheidet verschiedene Formen der Leberzirrhose. Bei der inaktiven, kompensierten Form liegt nur eine mäßige Leberzellinsuffizienz vor. Die aktive Form ist durch akut dystrophische Schübe oder eine schwere Leberzellinsuffizienz bis hin zum Präkoma oder Koma charakterisiert. Bei der schwersten Form, der dekompensierten Zirrhose beobachtet man Pfortaderhochdruck, Ösophagusvarizen und Ascites. Im Endstadium mit dem Zusammenbruch der Leberfunktionen tritt der Tod meist durch Verbluten aus Ösophagusvarizen oder im Koma hepatikum ein.

Die modernen Behandlungsmethoden der Lebererkrankungen und besonders des Endstadiums der Leberzirrhose haben dazu geführt, daß in den letzten Jahren die Lebenserwartung der Patienten immer weiter gestiegen ist und immer mehr der Patienten mit schwerem Leberparenchymschaden das finale Stadium der portalen Hypertension erreichen. Bei dieser Pfortader-Stauung entsteht ein erhöhter Druck im Pfortadersystem infolge mechanischer Strömungshindernisse in der Leber. Als Folge kommt es dann zu den klinischen Symptomen der Ösophagusvarizen-Blutungen und der portalen Enzephalopathie.

Während man die Blutungen aus den Ösophagusvarizen chirurgisch beherrschen kann und ebenfalls mit chirurgischen Maßnahmen den Pfortaderhochdruck durch Legen von Shunts entlasten kann, wird durch diese Vorgehensweise die portale Enzephalopathie verstärkt.

Bei der portalen Enzephalopathie kommt es auf Grund der Intoxikation mit Ammoniak und anderen Abbauprodukten aus Proteinen zu neurologischen und psychopathologischen Störungen, die sich unter anderem in Depressionen, Müdigkeit, Antriebsschwäche, Bewußtseinsstörungen bis hin zur Bewußtlosigkeit, dem hepatischen Koma äußern. 30 bis 40 % der Leberzirrhose-Kranken sterben im Koma hepatikum.

Normalerweise werden beim Menschen mit intakter Leberfunktion das im Darm bei der Verdauung entstehende Ammoniak und andere Proteinabbauprodukte in der Leber entgiftet. Wenn sich wegen der portalen Hypertension spontan ein Umgebungskreislauf gebildet hat oder chirurgisch als Shunt ausgebildet wurde, gelangen diese Giftstoffe unter Umgehung der Leber direkt in den Kreislauf und damit direkt in das Gehirn. Die resultierenden Ausfallerscheinungen werden als exogenes Leberausfallskoma bezeichnet.

Das endogene Leberzerfallskoma ist durch ausgedehnte Lebernekrosen mit Verlust der natürlichen Entgiftungsfunktion z.B. nach Virushepatitiden oder Vergiftungen gekennzeichnet.

Während der Ammoniakspiegel im Gesunden nur ca. 30 bis 80 µg/100 ml Plasma beträgt, reichert sich dieser Giftstoff bei Leberzirrhose-Kranken auf bis zu 500 µg/100 ml im Plasma an. In solch hohen Konzentrationen, die bisher kaum einer Therapiemöglichkeit zugänglich waren, beobachtet man bereits die schwersten neurologischen Ausfallserscheinungen.

Eine kausale Therapie der meisten chronischen Lebererkrankungen ist nicht möglich, sondern man beschränkt sich bei den Therapieplänen im wesentlichen auf die Umstellung der Ernährung und das Weglassen schädigender Noxen (z.B. Alkohol). Sogenannte Leberschutzpräparate sollen die Leber in ihrer restlichen Stoffwechselfunktion entlasten, damit sie ihre Entgiftungsfunktion für den Organismus so weit wie noch möglich wahrnehmen kann.

Zu den Leberschutzpräparaten zählen z.B. das Kohlehydrat Lactulose oder Milch mit lebenden Lactobacillus bifidus Bakterien, die die Bildung von Ammoniak durch Darmbakterien zurückdrängen und so die Leber entlasten sollen. Nebenwirkungen dieser diätätischen Maßnahmen sind Blähungen und Durchfälle. Auch der hohe Kohlehydratgehalt der Lactulose kann ein Problem darstellen.

Als weitere diätätische Maßnahme wird versucht, durch Gabe der Ammoniak-senkenden Aminosäuren Ornithin und Aspartat in den Harnstoff- und Citatzyklus einzugreifen und die Beseitigung des als Stoffwechselprodukt gebildeten Ammoniaks zu unterstützen. Auch die Aminosäuren Asparagin- und Glutaminsäure können Ammoniak durch Amidierung entgiften. Diese Therapie mit Aminosäuren ist aber nur im Frühstadium der Leberschädigung aussichtsreich.

Zur Verminderung der Entstehung von Ammoniak bei Leberschäden wird außerdem versucht, im Rahmen der Ernährung auf die Aminosäuren Threonin, Serin, Tryptophan, Histidin, Glutamin und Glycin weitgehend zu verzichten bzw. die Aufnahme der essentiellen Aminosäuren Threonin und Histidin auf das Nötigste zu beschränken, da diese Aminosäuren bis zu Ammoniak abgebaut werden und den Ammoniakspiegel im Plasma erhöhen.

Die diätätische Beeinflussung durch Aminosäuren und Proteine ist stets ein Balanceakt zwischen der Zufuhr von Stickstoff mit dem folgenden potentiellen Anstieg des Ammoniaks und der Notwendigkeit, die Patienten mit essentiellen Aminosäuren zu versorgen. Die Patienten sind meist auf eine hochkalorische Ernährung angewiesen, zu der auch Proteine gehören sollten. Leberzirrhose-Patienten werden deshalb so lange wie möglich besonders Eiweiß-reich ernährt, wobei besonders Quark (Casein aus Milch) bevorzugt wird. Da aber bei diesen Patienten die Eiweiß-Toleranz bei fortschreitenden Parenchymschäden immer stärker herabgesetzt ist, führt dies wiederum zum Anstieg des Ammoniakspiegels im Plasma. So führt z.B. die Aufnahme von normaler Milch (0,5 g Milchpulver pro kg KGW) zu einer Zunahme des Blutammoniakspiegels im Gesunden und im Zirrhose-Kranken (Gorski, M et al. Pol. Arch. Med. Wewn 1970, 44 (2), 115-9).

Um so überraschender war der Befund, daß man mit der Gabe von etwa der gleichen Menge Protein aus Kolostralmilch vom Rind bzw. mit der Molke aus dieser Kolostralmilch erreichen kann, daß der Blutammoniakspiegel bei Patienten mit Leberzirrhose drastisch sinkt. Die Abnahme des Ammoniaks geht teilweise soweit, daß das natürliche Niveau noch deutlich unterschritten wird.

Unter Kolostralmilch versteht man die Milch der ersten fünf Tage nach dem Kalben. Bevorzugt wird die Kolostralmilch von den ersten drei Tagen und besonders bevorzugt vom ersten Tag nach dem Kalben, da hier der Gehalt an Immunglobulinen am höchsten ist und den Antikörpern in der Kolostralmilch eine besondere Bedeutung für ihre Wirksamkeit zukommt.

Der therapeutische Einsatz von Kolostralmilch bzw. von Proteinfraktionen aus Kolostralmilch hat sich bisher im wesentlichen auf die Therapie oder Prophylaxe von durch Bakterien, Viren oder Protozoen, wie z.B. E. Coli, Rotavirus oder Cryptosporidien hervorgerufenen Durchfallerkrankungen beschränkt. Beispiele sind bei Tacket, C et al., The New England Journal of Medicin (1988), 318, 1240-12043, bei Davidson, G. et al., The Lancet 23.9.1989, 709-712 und bei Rump, J. et al., Clin. Investig. (1992), 70, 588-594 beschrieben worden.

Von Prokopiv, M., Vrach Delo 4, 100-102 (1988) wurde gezeigt, daß bei Patienten mit Multipler Sklerose, einer entzündlichen Erkrankung des zentralen Nervensystems, durch tägliche Gabe von 2 mal 500 ml humanen Kolostrums über 6 Wochen der Plasmaspiegel der Leberenzyme Alanin-Aminotransferase, Aspartat-Aminotransferase und Sorbit-Dehydrogenase leicht reduziert werden kann. Bei diesen Patienten gab es keine Hinweise auf eine Lebererkrankung, sondern diese membranständigen Leberenzyme wurden auf Grund der erhöhten Membranpermeabilität, die man generell bei Multipler Sklerose vorfindet, in das Plasma abgegeben. Das humane Kolostrum, das sich im Übrigen in seiner Zusammensetzung stark von bovinem Kolostrum unterscheidet, eignet sich danach für unterstützende Therapiemaßnahmen zur Hemmung der Membrandefekte bei Multipler Sklerose. Eine therapeutische Wirkung der Rinder-Kolostralmilch bei Lebererkrankungen, bei denen es nicht nur zu einer Erhöhung der Membranpermeabilität, sondern zu massiver Zelldegeneration und -Zerfall kommt, und insbesondere gegen die toxischen Folgen der Proteinabbauprodukte wurde bisher nicht beschrieben.

Überraschend und bisher nicht bekannt war aber nun der Befund, daß durch eine orale oder enterale Gabe von Kolostralmilch vom Rind die Verminderung der toxischen Protein-Stoffwechselprodukte wie z. B. Ammoniak möglich ist und die Entgiftungsfunktion der Leber für diese Stoffe verbessert werden kann.

Dies ermöglicht, die Patienten mit großen Mengen Protein, und damit mit essentiellen Aminosäuren, zu versorgen, ohne die schädlichen Wirkungen dieser Proteinzufuhr, wie z.B. das massive Auftreten von Proteinabbauprodukten wie z.B. Ammoniak, im Blut befürchten zu müssen.

Die erfindungsgemäße Verwendung wird vorzugsweise mit einer Rinder-Kolostralmagermilch oder mit einer Kolostralmolke durchgeführt, wie sie z.B. in den Patentanmeldungen EP 0 338 229 und EP 471 890 beschrieben werden.

Um eine optimale Schutzwirkung auszuüben, sollten die Präparate keimfrei oder zumindestens keimarm sein. Anderenfalls könnte durch eine Verunreinigung der Kolostralmilch mit Bakterien und dabei freigesetzten Giftstoffen der therapeutische Effekt aufgehoben werden.

Bei der Sterilisation und Entkeimung der Kolostralmilch muß auf die Nativität der Proteine in der Kolostralmilch geachtet werden, da nur die intakten und nativen Proteine die Schutzwirkung auf die Leber entfalten können. Vorzugsweise wird die Entkeimung durch eine Sterilfiltration erreicht, da hierbei die Bakterien zuverlässig abgetrennt werden, ohne daß die Gefahr der Freisetzung von Giftstoffen aus den Bakterien besteht.

Gegebenenfalls kann man eine Kolostralmolke durch weitere Fraktionierung aufreinigen, in dem man zum Beispiel niedermolekulare Proteine und andere Bestandteile mit einem Molekulargewicht kleiner als 100.000 D durch Ultrafiltration entfernt.

Die Behandlung der Patienten wird vorzugsweise mehrmals am Tag durchgeführt, wobei die Kolostralmilch-Präparate als Lösung mit einem Proteingehalt von 2 g/100 ml bis 20 g/100 ml, vorzugsweise 10 g/100 ml getrunken wird. In besonderen Fällen kann auch eine Applikation über eine Magen- oder Duodenalsonde erfolgen. Als therapeutische Dosis bei einer schweren Leberzirrhose sind 100 bis 600 ml einer 10 % igen Lösung pro Tag meistens ausreichend, während bei einer leichteren Verlaufsform auch geringere Mengen gegeben werden können.

Bei den Lebererkrankungen, deren Folgen erfindungsgemäß behandelt werden können, handelt es sich um Leberentzündung, Virus-Hepatitis, toxische Leberzellschäden, Leberfibrose, Leberzirrhose, Stauungsleber, Leberdystrophie, fettige Degeneration der Leberzellen oder Fettleber. Die aus diesen Erkrankungen sich ergebenden Störungen und Schäden, deren Folgen behandelt werden können, sind Störungen der Entgiftungsfunktion, Störungen der exkretorischen Funktion der Leber, Störungen der Konjugationsfunktion der Leber, Störungen der Synthesefunktion der Leber, durch eine Lebererkrankung bedingte portale Hypertension oder ein Leberausfallkoma sowie Intoxikationen durch Proteinabbauprodukte oder Ammoniak. So können z.B. Intoxikation durch Ammoniak bei Zustand nach portaler Shunt-Operation, wie z.B. bei portokavalen Anastomosen, behandelt werden.

Die erfindungsgemäße Verwendung des Kolostralmilchpräparates ist auch prophylaktisch möglich. Insbesondere bei Virus-Hepatiden und bei beginnender Leberinsuffizienz auf Grund von Leberparenchymschäden wird die Leber entlastet und die Entgiftungsfunktion durch Verbesserung der Konjugations- und der Exkretions-Funktion der Leber verbessert, so daß die nekrotischen Veränderungen der Leber verlangsamt werden. Diagnostisch äußert sich dies unter anderem in einer deutlichen Reduktion des Plasmaspiegels von Bilirubin und des Leberenzyms Gamma-Glutamyltransferase (GGT), einem intrazellulären Enzym, das bei dem Stoffwechsel von Aminosäuren und Peptiden eine wichtige Rolle spielt und das beim Leberzellzerfall in das Plasma übertritt. Außerdem findet man eine Steigerung der Synthese von Plasmaproteinen, wie z.B. Transferrin. Auf diese Weise kann das finale Stadium der portalen Enzephalopathie hinausgezögert werden.

In gleicher Weise lassen sich Lebererkrankungen wie Leberfibrose, die Stauungsleber, die Leberdystrophie und die fettige Degeneration der Leber behandeln. Durch die erfindungsgemäße Verwendung der Kolostralmilchpräparation wird die Leber entlastet.

In der Regel geht die Verzögerung bzw. Vermeidung des Anstiegs des Blutammoniak-Gehaltes mit einer Besserung oder Vermeidung der neurologischen Ausfallserscheinungen einher.

Durch die entsprechende prophylaktische Behandlung von Patienten mit vorgeschädigter Leber kann ein Anstieg des Blutammoniaks vermieden werden, wodurch sich diese Ausfallserscheinungen verhindern lassen.

Auch zur Vorbehandlung und Nachbehandlung bei Lebertransplantationen ist der Einsatz der Rinder-Kolostralmilch als Leberschutzpräparat möglich, um die Entgiftungsfunktion der geschädigten Leber vor der Transplantation, bzw. die neue Leber nach der Transplantation von schädlichen Stoffwechselprodukten zu entlasten.

Die folgenden Beispiele erläutern die Erfindung.

### Beispiel 1

Ein 45 Jahre alter Mann, der unter einer Leberzirrhose mit portaler Hypertension litt, wurde wegen einer schweren Oesophagusvarizenblutung stationär aufgenommen. Als Folge des mit den Blutungen verbundenen Kreislaufschockes entwickelte sich bei dem Patienten ein Leberversagen. Der Blut-Ammoniakspiegel stieg auf 170 µg/dl. Der Patient zeigte Symptome einer portalen Enzephalopathie.

Der Patient erhielt nun über eine Magensonde täglich 4 mal 15g einer gefriergetrockneten Rinder-Kolostralmolkepräparation. Das Präparat wurde aus Rinder-Kolostralmilch aus dem ersten Gemelk nach dem Kalben durch Entfetten mittels Zentrifugation, Caseinfällung mit Salzsäure bei pH 4,5, Ultrafiltration und Sterilfiltration der Molke und anschließender Trocknung gewonnen. Das Präparat wurde als 10 %ige Lösung in 0,9 % Kochsalzlösung appliziert. Innerhalb von 24 Stunden sank der Blut-Ammoniak-Spiegel auf 10 µg/dl und blieb im Verlauf der fünf-tägigen Therapie im Normbereich (30 bis 80 µg/dl).

Der Zustand des Patienten besserte sich unter der Kolostralmilch-Protein-Gabe zusehends. Der Patient zeigte bereits nach einem Tag keine neurologischen Ausfallerscheinungen mehr und war normal ansprechbar. Der Blut-Ammoniak blieb unter der Therapie mit der Kolostralmilch-Präparation niedrig.

Nebenwirkungen traten nicht auf. Dies war besonders überraschend unter Berücksichtigung der klinischen Erfahrungen, daß bei diesem Patienten-Typ normalerweise eine verminderte Eiweißtoleranz mit Anstieg des Ammoniakspiegels zu beobachten ist.

In einer Kontrollgruppe aus 21 Patienten mit gleichem Krankheitsbild, die kein Kolostralmilch-Präparat bekommen hatten und die dagegen auf herkömmliche Art mit Lactulose behandelt wurden, wurde über den gesamten Beobachtungszeitraum ein gleichbleibend hoher Ammoniak-Spiegel von 200 bis 300 µg/dl gefunden.

### Beispiel 2

Ein 58 Jahre alter Mann mit einer Leberzirrhose wurde ebenfalls wegen einer schweren Oesophagusvarizenblutung behandelt. Er entwickelte ähnlich wie in Beispiel 1 als Folge der Blutungen einen haemorrhagischen Schock mit Leberversagen. Der Blut-Ammoniakspiegel lag unmittelbar nach der stationären Aufnahme bei 270 µg/dl.

Unter der täglichen Therapie mit 3 mal 15 g des gleichen Kolostralmilchpräparates wie in Beispiel 1 sank der Ammoniak-Gehalt des Blutes auf 90 µg/dl und blieb auf diesem nur noch leicht über dem Normbereich liegenden Niveau im Verlauf der vier-tägigen Therapie.

### Beispiel 3

Bei einer 50 Jahre alte Frau, bei der sich auf Grund einer Leberzirrhose postoperativ ein schweres Leberversagen entwickelt hatte, wurde zu diesem Zeitpunkt ein stark erhöhter Blutammoniakspiegel von 350 µg/ml gemessen.

Die Patientin erhielt 3 mal 15 g der Kolostralmilchpräparation aus Beispiel 1 pro Tag über fünf Tage. Unter der Therapie wurde eine deutliche Abnahme des Ammoniaks auf 120 µg/100 ml innerhalb von 24 Stunden beobachtet. Die klinischen Zeichen der Leberfunktionsstörung wurde unter der Kolostralmilchtherapie positiv beeinflußt und der vor der Therapie sehr schlechte geistige Zustand der Patientin besserte sich rasch unter der Gabe der Kolostralmilchpräparation.

### Beispiel 4

Bei einem 48 Jahre alten Mann entwickelte sich 3 Tage nach einem operativen Eingriff eine Leberinsuffizienz bei bestehender Leberzirrhose mit erhöhten Ammoniak-Werten im Plasma.

Der Patient erhielt über eine Magensonde 4 mal 15 g der Kolostralmilchpräparation aus Beispiel 1 pro Tag über vier Tage. Unter der Therapie wurde bereits innerhalb von 17 Stunden eine rasche und deutliche Abnahme des Ammoniaks von 254 µg/100 ml auf 126 µg/100 ml beobachtet. Nach vier Tagen Therapie mit dem Kolostralmilch-Präparat betrug der Blutammoniak-Spiegel 108 µg/100 ml. Die klinischen Zeichen der Leberfunktionsstörung wurde unter der Kolostralmilchtherapie positiv beeinflußt. Nach Absetzen des Kolostralmilch-Präparates stieg der Blutammoniak innerhalb von zwei Tagen wieder auf 250 µg/100 ml an.

Dieser Auslaßversuch zeigt durch die auf den Zeitpunkt der Therapie begrenzte Senkung des Blutammoniakspiegels eindrucksvoll die Wirksamkeit der Therapie mit Rinder-Kolostralmilch.

### Beispiel 5

Bei einer 44 Jahre alten Patientin entwickelte sich postoperativ eine Leberinsuffizienz auf der Basis einer bestehenden Leberzirrhose.

Vor sowie am 1. und am 3. Tag der 3-tägigen Therapie mit 3 mal 20 g der Kolostralmolkepräparation analog Beispiel 1, gelöst in jeweils 200 ml Wasser, wurden die Laborparameter bestimmt, die in Tabelle 1 wiedergegeben sind.

**Tabelle 1**

| | vor Therapie | nach 24 Std. | nach 72 Std. |
|---|---|---|---|
| GGT (U/l) | 105 | 97 | 78 |
| Transferrin (mg/100 ml) | 119 | 140 | 152 |

Die GGT sinkt unter der Therapie, während die Syntheseleistung der Leber wieder ansteigt, wie an der Zunahme des Transferrinspiegels gesehen werden kann.

### Beispiel 6

Ein 9 Jahre alter Junge hatte wegen einer Hepatitis pathologisch erhöhte GGT-Werte im Plasma.

Nach Gabe von täglich 2 mal 100 ml einer 10 %-igen Kolostralmolkelösung analog Beispiel 1 sanken die Werte für dieses Leberenzym innerhalb von wenigen Tagen von 53 auf 21 U/ml für die GGT und blieben unter der Therapie in diesem Bereich relativ konstant.

Nach Absetzen des Präparates nach 10 Wochen stiegen die Werte dann wieder auf 68 U/l.

## Patentansprüche

1. Verwendung einer unter keimarmen Bedingungen gewonnenen bovinen Kolostralmilch mit nativen Proteinen zur Herstellung eines Arzneimittels für die Behandlung von Folgen der Leberfunktionsstörungen und/oder Leberparenchymschäden bei Erkrankungen der Leber.

2. Verwendung gemäss Anspruch 1, dadurch gekennzeichnet, daß die Kolostralmilch in den ersten 5 Tagen nach dem Kalben gewonnen wird.

3. Verwendung gemäss Anspruch 1 oder 2, dadurch gekennzeichnet, daß die Kolostralmilch in den ersten 3 Tagen nach dem Halben gewonnen wird.

4. Verwendung gemäss einem der Ansprüche 1 bis 3, dadurch gekennzeichnet, daß die Kolostralmilch in den ersten 24 Stunden nach dem Kalben gewonnen wird.

5. Verwendung gemäss einem der Ansprüche 1 bis 4, dadurch gekennzeichnet, daß die Kolostralmilch-Zubereitung weitgehend frei von Bakterien und Toxinen ist.

6. Verwendung gemäss einem der Ansprüche 1 bis 5, dadurch gekennzeichnet, daß die Kolostralmilch entfettet wurde.

7. Verwendung gemäss einem der Ansprüche 1 bis 6, dadurch gekennzeichnet, daß die Kolostralmilch entfettet wurde und daß das Casein weitgehend abgetrennt wurde.

8. Verwendung gemäss Anspruch 7, dadurch gekennzeichnet, daß die Kolostralmilch entfettet, das Casein weitgehend abgetrennt und Proteine mit einem Molekulargewicht kleiner als 100 000 D weitgehend abgetrennt wurden.

9. Verwendung nach einem der Ansprüche 1 bis 8, dadurch gekennzeichnet, daß die Kolostralmilch-Zubereitung abschließend sterilfiltriert wird.

## Claims

1. Use of bovine colostrum with native proteins obtained under hygienically clean conditions for the production of a pharmaceutical preparation for the treatment of the consequences of disrupted liver function and/or damage to the hepatic parenchyma in liver disease.

2. Use according to claim 1, characterised in that the colostrum is obtained in the first 5 days after calving.

3. Use according to claim 1 or 2, characterised in that the colostrum is obtained in the first 3 days after calving.

4. Use according to one of claims 1 to 3, characterised in that the colostrum is obtained in the first 24 hours after calving.

5. Use according to one of claims 1 to 4, characterised in that the colostrum preparation is largely free of bacteria and toxins.

6. Use according to one of claims 1 to 5, characterised in that the colostrum has been defatted.

7. Use according to one of claims 1 to 6, characterised in that the colostrum has been defatted and that the casein has largely been separated.

8. Use according to claim 7, characterised in that the colostrum has been defatted, the casein largely separated and proteins having a molecular weight of less than 100000 D have largely been separated.

9. Use according to one of claims 1 to 8, characterised in that the colostrum preparation is subsequently sterile-filtered.

## Revendications

1. Utilisation d'un colostrum bovin recueilli dans des conditions aseptiques avec des protéines natives pour la fabrication d'un médicament pour le traitement des suites des perturbations fonctionnelles du foie et pour des dommages de parenchyme du foie dus aux maladies du foie.

2. Utilisation selon la revendication 1, caractérisée en ce que le colostrum est recueilli dans les cinq premiers jours après le vêlage.

3. Utilisation selon la revendication 1 ou 2, caractérisée en ce que le colostrum est recueilli dans les trois premiers jours après le vêlage.

4. Utilisation selon l'une des revendications 1 à 3, caractérisée en ce que le colostrum est recueilli dans les 24 premières heures après le vêlage.

5. Utilisation selon l'une des revendications 1 à 4, caractérisée en ce que la préparation de colostrum est exempte dans une large mesure de bactéries et de toxines.

6. Utilisation selon l'une des revendications 1 à 5, caractérisée en ce que le colostrum est dégraissé.

7. Utilisation selon l'une des revendications 1 à 6, caractérisée en ce que le colostrum est dégraissé et la caséine est séparée dans une large mesure.

8. Utilisation selon la revendication 7, caractérisée en ce que le colostrum est dégraissé, la caséine est séparée dans une large mesure et les protéines ayant une masse moléculaire inférieure à 100 000 D sont séparées dans une large mesure.

9. Utilisation selon les revendications 1 à 8, caractérisée en ce que la préparation de colostrum est finalement filtrée stérilement.
